# EUROPEAN PATENT SPECIFICATION

(11) **EP 1 844 072 B1**
(45) Date of publication and mention of the grant of the patent: **04.04.2012**
(21) Application number: 06703945.3
(22) Date of filing: 01.02.2006
(51) Int. Cl.: C07K 14/705, G01N 33/569

(54) **MHC OLIGOMER AND METHOD OF MAKING THE SAME**
MHC-OLIGOMER UND VERFAHREN ZU DESSEN HERSTELLUNG
OLIGOMERE MHC ET PROCEDE DE FABRICATION DE CELUI-CI

(30) Priority: 04.02.2005 GB 0502333
(43) Date of publication of application: 17.10.2007
(73) Proprietor: ProImmune Limited, Oxford OX4 4GA (GB)
(72) Inventor: SCHWABE, Nikolai, Franz, Gregor, Oxford OX2 6BP (GB)
(74) Representative: Vossius & Partner
(86) International application number: PCT/GB2006/000320
(87) International publication number: WO 2006/082387

(56) References cited:
- WO-A-98/05684
- WO-A-2004/018520
- BONIFACE J J ET AL: "pH affects both the mechanism and the specificity of peptide binding to a class II major histocompatibility complex molecule." BIOCHEMISTRY. 9 NOV 1993, vol. 32, no. 44, 9 November 1993 (1993-11-09), pages 11761-11768, XP002375627 ISSN: 0006-2960
- KITA HIROTO ET AL: "Application of tetramer technology in studies on autoimmune diseases." AUTOIMMUNITY REVIEWS. JAN 2003, vol. 2, no. 1, January 2003 (2003-01), pages 43-49, XP002375628 ISSN: 1568-9972
- SEOK-KI CHOI: "Synthetic Multivalent Molecules" 2004, JOHN WILEY & SONS, INC. , HOBOKEN, NEW JERSEY , XP001237256 ISBN: 0-471-56347-1 Introduction page 4 - page 7; figures 1.2,1.3
- LI YOUGEN ET AL: "Controlled assembly of dendrimer-like DNA." NATURE MATERIALS, vol. 3, no. 1, January 2004 (2004-01), pages 38-42, XP002375629 ISSN: 1476-1122

## Description

The present invention relates to an MHC oligomer comprising at least two functional MHC complexes having a peptide binding groove, each MHC complex having a peptide bound in the peptide binding groove of the MHC complex, wherein each peptide has a modification which allows, in a predetermined manner, specific and/or selective formation of an oligomer of the functional MHC complexes through a core structure, said core structure being an entity allowing a simultaneous binding of each peptide.

### Background of the Invention

Major Histocompatibility Complex (MHC) molecules, which are found on the cell surface in tissues, play an important role in presenting cellular antigens in the form of short linear peptides to T cells by interacting with T cell receptors (TCRs) present on the surface of T cells. They consist of alpha and beta chains, and a peptide bound in a groove formed by these chains when properly folded.

It has been established that isolated or recombinant forms of MHC-peptide molecules are useful for detecting, separating and manipulating T cells according to the specific peptide antigens these T cells recognise. It has also been understood that the interaction between MHC molecules and TCRs across cell surfaces is multimeric in nature and that the affinity of a single MHC molecule for a given TCR is generally quite low.

As a consequence, there has been an effort to develop multimeric forms of isolated or recombinant MHC-peptide molecules that have an increased functional avidity in order to make such molecules more useful in the applications described above.

European Patent Application EP 812 331 discloses a multimeric binding complex for labeling, detecting and separating mammalian T cells according to their antigen receptor specificity, the complex having the formula (α-β-P)ₙ, wherein (α-β-P) is an MHC peptide molecule, n is ≥ 2, α comprises an α chain of a MHC I or MHC II class molecule, β comprises a β chain of an MHC protein and P is a substantially homogeneous peptide antigen. The MHC peptide molecule is multimerised by biotinylating the C terminus of one of the α or β chain of the MHC molecule and coupling of MHC monomers to tetravalent streptavidin/avidin or by providing a chimeric protein of an MHC molecule which is modified at the C terminus of one of the a or β chain to comprise an epitope which is recognised by a corresponding antibody that serves as a multimerising entity. The document further teaches use of the MHC oligomers for detecting, labeling and separating specific T cells according to their TCR specificity.

WO 93/10220 discloses a chimeric MHC molecule, comprising the soluble part of an MHC molecule, which can be either class I or class II MHC fused to an immunoglobulin constant region. The MHC portion of the molecule comprises complementary a and/or β chains and a peptide is bound in the respective binding grooves of the MHC molecules. Due to the presence of the dimeric immunoglobulin scaffold these chimeric MHC-Ig molecules undergo self-assembly into a dimeric structure.

European Patent Application EP 665 289 discloses specific peptides, MHC molecules binding these peptides, and oligomers obtained by crosslinking of the respective MHC molecules having the specific peptide bound to them. Oligomerisation is achieved by using chemical crosslinking agents or by providing MHC chimeric proteins comprising an epitope, which is recognised by an immunoglobulin such as IgG or IgM. The MHC molecules may comprise a label and may be used labeling, detecting, and separating T cells according to their specific receptor binding, and may eventually be employed in therapy of humans.

In an alternative embodiment EP 665 289 describes oligomeric MHC complexes, which are oligomerised by using an oligomerised form of the MHC binding peptides. The oligomeric peptides may be linked through chemical modifications on the peptide or the oligomeric peptide may already form a linear oligomer, i.e. one peptide chain having several MHC binding portions.

US 2002/0058787 discloses peptide oligomers comprising at least two MHC binding peptides joined by a flexible molecular linker. The MHC binding peptides can be MHC class I binding peptides or MHC class II binding peptides. Also disclosed is an oriented cloning method for producing such oligomers. The disclosed oligomers can be used, for example, in connection with methods for specifically activating or inhibiting the activation of CD4+ or CD8+ T cells. Such methods provide therapeutic approaches for the treatment of tumours, autoimmune disorders, allograft rejection and allergic reactions: These peptide oligomers would however not be well suited for forming isolated MHC-peptide multimers, since it is usually necessary to incubate soluble MHC complexes or chains thereof in the presence of a molar excess of peptide. This would lead to very incomplete oligomerisation of the complexes and a situation where multiple sections of MHC-binding peptide in the resulting complexes are not bound to an MHC-peptide complex, which in turn can lead to decreased specificity and higher background binding of such complexes.

When constructing MHC multimers it can be desirable to construct MHC peptide monomers first and then to multimerise these monomers by attaching them to a multivalent entity (for example, as described in US5,635,363) or to one another. However the method described in US 5,635,363 requires an epitope or site for specific attachment of the monomers to a multivalent entity on the alpha or beta chain of the MHC peptide complex. In fact not many convenient ways exist to provide such a specific attachment site.

The simplest way may be to provide an antibody binding epitope at the C-terminal end of the MHC molecule and then multimerising the molecule via one or more antibodies that are specific for that epitope. The drawback of this technique is that monomeric antibody epitope interactions are typically not as strong as would be desirable and the resulting molecule could be quite large if it is multimerised in a two-step process, e.g. by binding epitope specific antibodies first and isotype specific antibodies second to the resulting MHC antibody complexes.

Any chemical site-specific modification of a polypeptide that has been produced by recombinant protein expression is difficult as most known targeted coupling methods are specific to one or several amino acids. As a consequence several amino acids are usually modified at the same time, including those of the antigen peptide bound in the MHC molecule after a monomeric complex is formed. This has an uncontrollable effect on the ability of the complex to bind to its complementary T cell receptor successfully This holds the more so true for any random cross-linking process.

As an alternative, it has been suggested to oligomerize the MHC complexes using the biotin-streptavidin system (see e.g. US 5,635,363). This method requires the site directed enzymatic biotinylation of the MHC molecules near one of its carboxyl termini. An enzyme recognition peptide sequence of around 14 amino acids is fused to the C-terminus of one MHC peptide chain, which then allows for the complex to be biotinylated by using a biotinylating enzyme recognising this site. Biotinylation thus involves a substantial number of process steps, including several rounds of protein purification, and an enzymatic biotinylation reaction that can lead to significant loss of active MHC complexes. Further, controlling the biotinylation efficiency of monomeric MHC subunits and quality of the final multimeric product is difficult. For example, where a specific MHC complex comprising homogeneous peptides is to be synthesized and the synthesis yield is very low, protein losses in the biotinylation reaction and lower than 100% biotinylation efficiency can drive the yield of the finished product below an acceptable level.

This methodology also limits the multimerisation method for the biotinylated complex to binding it to avidin family proteins, such as streptavidin, whith tetramerises it or to cross-linked variants of such proteins. With cross-linked avidin family protein variants it is however difficult to control the valency of the complexes accurately. In situations where such a tetrameric or non-uniform valency multimer is not desirable or the use of streptavidin or molecules related to streptavidin is unwanted this method also has serious limitations.

It is the object of the invention to provide an improvement over the prior art by providing MHC oligomers that allow for any desired degree of oligomerisation. It is further an object of the invention to provide an oligomer, which can be made with reasonable yields, and in superior purity.

### Summary of the Invention.

To overcome the abovementioned and other disadvantages of the prior art and to solve the above objects the present invention thus provides the following:

An MHC oligomer comprising at least two functional MHC complexes having a peptide binding groove, each MHC complex having a peptide bound in the peptide binding groove of the MHC complex, wherein each peptide has a modification which allows, in a predetermined manner, highly specific and/or selective formation of an oligomer of the functional MHC complexes through a core structure, said core structure being an entity allowing a simultaneous binding of each peptide.

Preferably the MHC oligomer substantially does not contain sections of MHC-binding peptide that are not bound to an MHC-peptide complex.

Preferably substantially none of the amino-acid side chains of the MHC binding portion of the peptide and/or of the MHC alpha or beta chains in the MHC-peptide complexes comprised in the MHC oligomer have been modified in the process of oligomerisation.

In a first specific embodiment the invention relates to an MHC oligomer ,
as defined in the claims, wherein the MHC complexes have been oligomerised at their peptides after assembly of the functional
monomeric MHC complexes including the peptide.

In a second specific embodiment the invention relates to an MHC oligomer as defined in the claims,
wherein each peptide comprises a modification selected from the group consisting of a specific attachment site and an oligomerisation domain,
wherein oligomerisation of the functional MHC complexes occurs through
(i) binding of each peptide to a multivalent entity at the specific attachment site provided on or attached to each peptide, or
(ii) alignment of the peptides through the oligomerisation domain provided on or attached to each peptide
wherein the MHC complexes in the oligomer are connected to a core structure provided by the multivalent entity or by alignment of the peptides.

In a second aspect the invention also provides a pharmaceutical or diagnostic composition comprising an MHC oligomer as defined above, optionally in combination with a pharmaceutically acceptable carrier.

In a third aspect the present invention provides several methods of labeling and/or detecting and/or separating mammalian T cells according to the specificity of their antigen receptor capable of binding functional MHC complexes.

In a fourth aspect the present invention provides a method of forming an MHC oligomer as defined in the claims
comprising at least two functional MHC complexes having a peptide binding groove, each MHC complex having a peptide bound in the peptide binding groove of the MHC complex, wherein each peptide has a modification which allows highly specific oligomerisation of the functional MHC complexes through a core structure, said method comprising the steps of:
(i) providing one or more peptides capable of binding in the peptide groove of each functional MHC complex, said peptide being modified by
   (a) providing a specific attachment site; or
   (b) providing an oligomerisation domain;
(ii) providing monomeric functional MHC-peptide complexes including the peptide of (i);
   and
(iii) oligomerising the functional MHC complexes through
   (a) providing a multivalent entity and binding of the said monomeric functional MHC-peptide complexes to the multivalent entity at the specific attachment site provided on each peptide, or
   (b) alignment of the peptides through the oligomerisation domain provided on or attached to each peptide.

### Brief Description of the Drawings

Fig. 1 shows a monomeric class II MHC peptide complex, which comprises a modified peptide according to the invention suitable for subsequent multimerisation;
Fig. 2 shows a monomeric class I MHC peptide complex, which comprises a modified peptide according to the invention suitable for subsequent multimerisation;
Fig. 3 shows a branched peptide suitable for multimerising MHC complex monomers at their binding peptide according to the invention; and
Fig. 4 shows a cyclic oligonucleotide, suitable for multimerising MHC complex monomers at their binding peptide according to the invention.
Fig. 5 shows modified peptides that enable forming multimeric MHC complexes without a separate multivalent entity.

### Detailed Description of the Invention

The MHC oligomers of the present invention are oligmerised through the MHC binding peptide by oligomerising in a highly specific manner functional, peptide-containing monomeric MHC peptide complexes after they have been assembled through a modification provided on the peptide. For oligomerisation the peptide contained in each monomeric MHC peptide complex is oligomerised in highly specific manner via the peptides to a core structure. To that purpose the peptides are either attached to a multivalent entity or the peptides self-assemble or oligomerise, thereby creating a core structure of the oligomer formed.

The term "core structure" as used herein is intended to designate an entity allowing a simultaneous binding of each peptide and hence the MHC monomers. The core structure may either be provided by a separate multimeric entity, to which the peptides are attached. In the alternative, the peptides themselves by oligomerisation may create such core structure. This includes the oligomerisation domain of the peptides, to which the MHC binding portion is bound and from which it is pending. In other words, the modification of the peptides allows them to self assemble or align, thereby creating a new core structure to which all peptides are bound simultaneously.

As used herein, the terms "multimerisation" or "oligomerisation" designate the phenomenon of creating an at least for a desired time stable complex comprising at least two functional MHC monomers. Both terms are considered exchangeable.

Based on the type of oligomerisation chosen the MHC oligomers of the present invention can have a well controllable predetermined valency and very high purity since the individual monomeric MHC-peptide complexes can be purified and assembled separately and multimerised subsequently. In addition, in some embodiments the invention has the advantage that monomeric MHC-peptide complexes can be synthesized and, if necessary, modified using well known synthesis methods. Multimerisation can take place after monomer synthesis without requiring the monomers to be further modified at that time, thus avoiding additional protein loss at that stage. Further, modifications for the purpose of subsequent multimerisation are only required to be made on the small peptide bound in the MHC peptide binding groove. This can, however, conveniently be achieved using, e.g. solid phase technology, which allows great flexibility for introducing site-specific modifications.

As a consequence, MHC oligomers formed according to the invention can be designed to have advantageous steric conformation as they are easily oriented in a planar configuration with all MHC peptide-binding faces in the complex facing T cell receptors on the surface of antigen-specific T cells. They can also be made with a precise and well controlled stoichiometry. In addition, the oligomers formed will be substantially free of peptide MHC-binding portions not bound to an MHC molecule.

Under the invention functional monomeric MHC complexes are formed first. This may either be by refolding of the relevant MHC alpha and beta chains from inclusion body material in the presence of the modified peptide of interest or by expression of native monomeric MHC peptide complexes in an eukaryotic expression system preferably in the presence of the modified peptide of interest. In both cases the refolding may also be followed by peptide exchange of with such modified peptide of interest.

Oligomerisation of the MHC complexes occurs after functional monomeric MHC peptide complexes have been formed through an appropriate reaction chemistry or mechanism enabled in part by the modification provided on the peptide. Typically, this will be carried out under physiological buffer conditions that do not disrupt or alter the binding properties of the monomeric MHC peptide complexes. The invention thus also provides for a high yielding method, which retains the functionality of the oligomerised MHC complex monomers.

For the avoidance of doubt the reference to monomeric MHC peptide complexes made herein does not exclude that such monomeric complexes are already pre-multimerised to a certain degree before further oligomerisation occurs at their peptide. E.g. monomeric MHC-peptide complexes may already be provided as MHC-Ig fusion dimers, such as described in WO9310220 before being oligomerised to become higher-valent MHC-peptide oligomers. Hence monomeric MHC peptide complexes as used herein merely refers to the fact that functional monomeric sub-units comprising the peptide of interest will be formed first before further oligomerisation occurs at the peptides. Preferably, however, the oligomeric MHC complexes of the invention will be generated by forming truly monomeric MHC-peptide complexes in the first instance.

In a first aspect the invention thus provides a MHC oligomer as defined in the claims comprising at least two functional MHC complexes having a peptide binding groove, each MHC complex having a peptide bound in the peptide binding groove of the MHC complex, wherein each peptide has a modification allowing, in a predetermined manner, specific and/or selective formation of an oligomer of the functional MHC complexes through a core structure, said core structure being an entity allowing a simultaneous binding of each peptide. The modification may preferably be selected from the group consisting of an attachment site or an oligomerisation domain.

Depending on the modification chosen, oligomerisation of the functional MHC complexes then occurs through (i) binding of each peptide to a multivalent entity at the specific attachment site provided on or attached to each peptide, or (ii) alignment of the peptides through the oligomerisation domain provided on or attached to each peptide. In any case the MHC complexes in the oligomer are connected to a core structure provided by the multivalent entity or by alignment of the peptides.

As used herein the term "highly specific" oligomerisation means a specificity and/or selectivity of forming the oligomer (by attachment or alignment), preferably at physiological conditions, in a predetermined manner that avoids any modification to be made in the oligomerisation reaction to the amino acids of the alpha or beta chain of the monomeric MHC-peptide complexes or the amino acids of the MHC binding portion of the MHC binding peptide, of preferably greater than 10 and more preferably greater than 100 and most preferably greater than 1000.

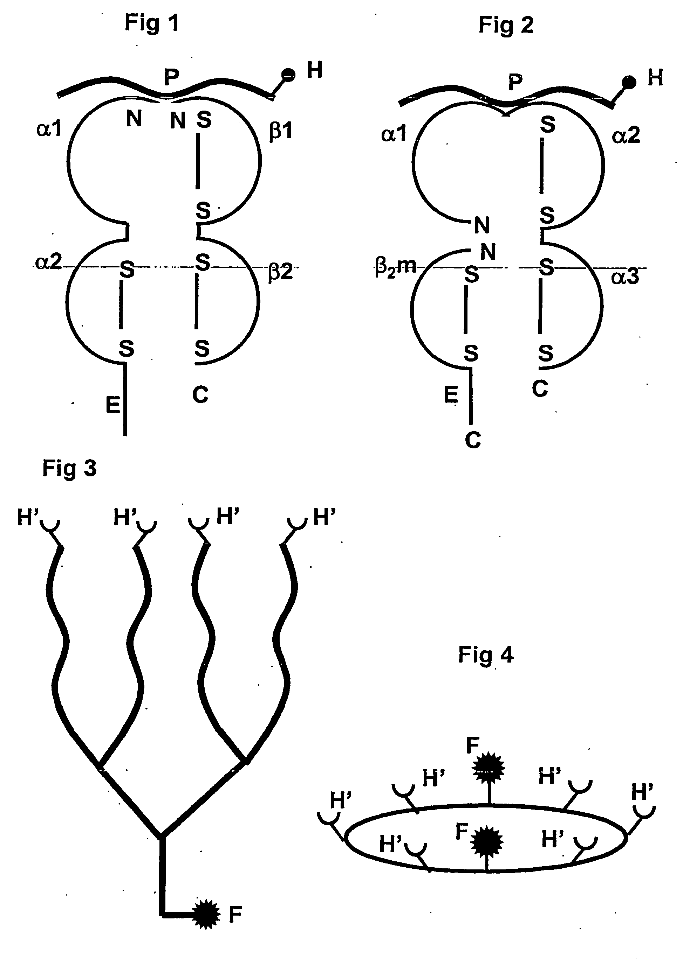

For illustration purposes a corresponding monomeric class II MHC complex is shown in Fig. 1 that has an antigenic peptide bound in its-groove. The figure shows the α1, α2 and β1, β2 domains of the MHC alpha and beta chains, respectively. The amino and carboxy termini of the respective polypeptide chains are labelled N and C respectively. The location of disulphide bonds is indicated by S-S. The MHC alpha chain is shown to have a tag domain (E). The antigenic or MHC binding portion of the peptide (P) is synthesized at one of its termini with a linker (not shown) and a modification (H) at the end of such linker in form of an attachment site or oligomerisation domain.

For Class II MHC binding peptides where it is known that binding peptides can extend out of the MHC binding groove the modification may be made either on or near the N or the C terminus of the peptide.

Fig. 2 shows a monomeric class I MHC complex that has a modified antigenic peptide bound in its groove, similar to Fig 1 (the figure is labelled with a nomenclature corresponding that of Fig 1). In the case of a class I MHC molecule the modification (H) in form of a specific attachment site or oligomerisation domain is preferably located at or near the C-terminus of the peptide, where the MHC binding peptide including the linker has more flexibility for overhanging the MHC binding groove than at the N-terminus.

In one embodiment oligomerisation occurs through attachment of the peptide to a multivalent entity. Such attachment may occur through recognition of a specific attachment site provided on or attached to the peptide by a complementary recognition site provided on the multivalent entity. The recognition site will allow for covalent or non-covalent binding of the peptide to the multivalent entity at the specific attachment site and the complementary recognition site, respectively. The valency of the multivalent entity will be determined by the number complementary recognition sites and their individual valency that are provided on the multivalent entity and that are accessible for binding.

If the attachment is non-covalent, the specific attachment site and the recognition site are selected from members of a specific binding pair. Suitable binding pairs of the necessary specificity are known in the art. Non-limiting examples of such binding pairs are hapten/antibody, epitope/antibody, ligand/receptor, substrate or substrate analogon/ enzyme, cofactor or cofactor analogon/enzyme, nucleic acid/complementary nucleic acid, sugar/lectin, biotin/avidin family protein, such as avidin, streptavidin, neutravidin, Streptag® / Streptactin®.

For example in case of the biotin/streptavidin binding pair, the biotin can be introduced into the MHC binding peptide at a pre-determined amino acid position through convenient chemical synthesis eliminating the need for cumbersome enzymatic biotinylation. Oligomerisation with streptavidin then results in a tetrameric MHC complex.

Alternatively the attachment of the peptide to the multivalent entity occurs through covalent binding of the peptide to the multivalent entity. In these cases the attachment site and the recognition site are complementary moieties capable of creating a covalent bond or coupling with each other, preferably under physiological conditions. Specific coupling approaches include, but are not limited to, the following types of covalent bonds: (a) oxime, (b) hydrazone, (c) thiazolidine, (d) oxazolidine, (e) thioether, (f) disulfide, and (g) peptide. Several chemistries known in the art are available to form such types of bonds.

In a preferred embodiment the moiety capable of creating a covalent bond or coupling is different from (i) a natural amino acid side chain and/or (ii) an amino acid carboxyl or amino terminal group and (iii) a combination of (i) and (ii).

In an alternative preferred embodiment an N-terminal cysteine is introduced into the peptide since the combination of the amino terminus and the thiol group of the cysteine can be used to bind selectively to a complementary thioester moiety to form a peptide bond, a complementary maleimide moiety to form a thioether bond, or to a complementary aldehyde moiety to form a thiazolidine bond. With the maleimide chemistry it is also possible to introduce the cysteine near the N-terminus, rather than at the N-terminus, if preferred, or at or near the C-terminus of the peptide

In a more preferable embodiment any one of the more recently developed specific chemoselective ligation chemistries using thiol chemistry or carbonyl chemistry are applied. For further details reference should be made to J.P. Tam and Y.A. Lu in "Chemoselective and orthogonal ligation techniques" in chapter 11 of Weng C. Chan and Peter D. White Eds., "Fmoc solid phase peptide synthesis, A Practical Approach" Oxford University Press (2000), which is incorporated herein by reference. Commercially available specific cross-linking tools, which can be applied in the present invention are e.g. available under the tradename HydraLinK® from EMD BioSciences, Inc., Darmstadt, Germany.

Characteristically such chemoselective ligation (i) uses unprotected peptide segments and (ii) the reaction is performed in aqueous conditions. To achieve these chemistries, a reactive pair consisting of a nucleophile and an electrophile is placed on the peptide P and the multivalent entity during their respective synthesis. Usually the nucleophile is a weak base, which has either a pKₐ significantly lower than the alpha- or epsilon-amines in natural amino acids or a nucleophilicity much stronger than such alpha-amines, so that the ligation can be selective in aqueous buffered solution at a pH of approximately 7. Cheomoselectivity is achieved when such mutually reactive groups are brought together in aqueous solution with the weak base as the sole nucleophile to react with the electrophile. Protection of other functional groups on the peptides involved therefore becomes unnecessary.

Generally it is preferable to use a chemoselective ligation chemistry that reacts complementary moieties provided as the specific attachment site on the MHC binding peptide and as the complementary recognition sites on the multivalent entity, whose reaction with one another can be carried out under environmental conditions such that none of the amino-acid side chains of naturally occurring amino acids will be modified in this reaction and that substantially do not ablate the functional integrity of native MHC-peptide complexes.

In a preferred embodiment the specific attachment site or the complementary recognition site will comprise an aldehyde group, which can be introduced into a polypeptide backbone as described in Tam and Lu *supra.* In a more preferred embodiment the other of the specific attachment site or the complementary recognition site will comprise a group that can react with an aldehyde group to form (a) oxime, (b) hydrazone, (c) thiazolidine, (d) oxazolidine, (e) thioether bond. In a most preferred embodiment the specific attachment site provided on the peptide (P) will comprise an aldehyde group.

Examples for the reaction chemistries forming bonds of the type (a) to (d) above are as follows:
(a) Oxime 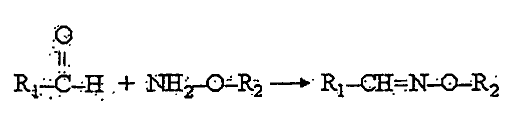
(b) Hydrazone 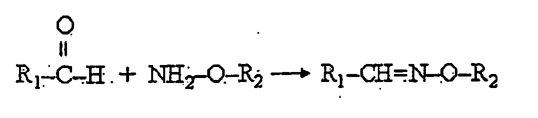
(c) Thiazolidine 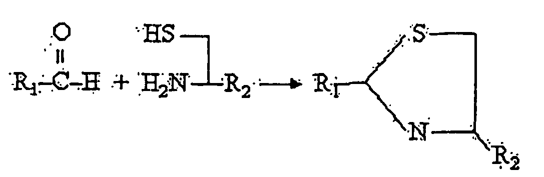
(d) Oxazolidine 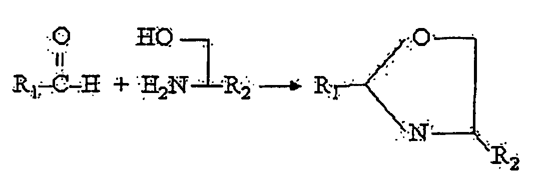
wherein R₁ is either one of (a) the unprotected modified peptide (P) or (b) the multivalent entity and R₂ is the respective other.

In each case the multivalent entity can be modified to incorporate a controlled number of copies of the recognition site. The system of complementary binding partners will have high specificity and selectivity. Each site may individually be monovalent or multivalent. In particular binding of the recognition site to the attachment site will be such that it occurs under conditions that do not impair the stability and activity of monomeric MHC peptide complexes, which means that it will typically be carried out under aqueous conditions and at near neutral pH.

It is also preferable that both sites are chosen such that the oligomerisation process does not modify any residue of the MHC binding portion of the peptide P or of the MHC alpha or beta chains.

Covalent coupling between the recognition site and the attachment site may be preferable in order to maximize the stability of the oligomeric MHC complex. For exploiting these covalent coupling chemistries, one of the necessary reactive moieties is provided at the attachment site of the peptide, whereas the other is provided at the recognition site on the multivalent entity. A simple approach of incorporating such reactive moieties is to incorporate a suitable modification at or near the amino or caboxy terminus of the multivalent entity and/or the peptide during or after peptide synthesis using the protocols as discussed by Tam and Lu (*supra*).

Chemoselective non-amide ligation will be preferable for coupling, as it is site specific and the reaction is performed in aqueous conditions at a pH of 7 or close enough to 7 not to interfere with the structure of native MHC-peptide complexes.

In a preferred embodiment the attachment site and the recognition site are 2-hydrazinopyridyl and benzaldehyde, respectively, which can be reacted optimally carried at pH4.7 to form bis-aromatic hydrazone. This reaction can also be carried out at pH up to 7.3, guaranteeing the stability of the MHC peptide monomers, although it occurs more slowly at higher pH. In order to combine speed of the reaction kinetics with conjugation conditions that do not impair the stability of MHC molecules a buffer pH of around 6.5-6.8 would be optimal.

In another preferred embodiment the attachment and recognition sites will be introduced into at least one of the peptide (P) and the multivalent entity by solid phase synthesis techniques. Several methods for introducing suitable groups are described in Tam and Lu (*supra*) as well.

It will be obvious to the skilled practitioner how to translate the above chemistries to a situation where the multivalent entity is not a polypeptide, but rather an oligonucleotide.

The multivalent entity used in the first and second alternative approach for oligomerisation according to the present invention can be any multivalent entity, as long as it does not unduly interfere with the T cell receptor binding of the MHC complex monomers. Valency of i.e. the number of recognition sites or reactive moieties on the multivalent entity and their spacing will determine the degree of oligomerisation. For example, a tetravalent entity such as e.g. streptavidin will result in a tetramer. Much higher valences are however possible. Preferably the valency of the entity will be in the range of 2 to 20, more preferably 4 to 10.

Preferably the multivalent entity is a natural polymer or a derivative thereof such as a protein, a branched polypeptide (dendrimer), a multimeric protein, a nucleic acid, a polysaccharide, such as dextran, starch, cellulose, hyaluronic acid, chitin, or alginic acid or a derivative of these polysaccharides, an oligonucleotide, a cyclic oligonucleotide; a synthetic polymer such as polypropyleneglycol, polyethyleneglycol (PEG); a phospholipid membrane, such as a vesicle or a liposome, and an inorganic particle e.g. polystyrene or acrylic beads or magnetic beads. The recognition site may be provided by the multivalent entity e.g. by its backbone, or may be attached thereto.

In a preferred embodiment the multivalent entity is a natural polymer (such as a protein or multimeric protein e.g. streptavidin, avidin, an immune globuline, hyaluronic acid, cellulose) or synthetic polymer (e.g. polyacrylic acid, polystyrene, polylactic acid) and the recognition sites are provided by the backbone of the multivalent entity, or are attached thereto.

In a more preferred embodiment the multivalent entity is a branched polypeptide (a so-called dendrimer). These dendrimers may for example be made according to the protocol as disclosed in Tam and Lu (*supra*). Several other methods for synthesizing branched polypeptides will be well known to the practitioner skilled in the art.

Preferably the branched polypeptide has specific recognition sites or members of a specific binding pair incorporated at predetermined sites in two or more of its branches. Each branch of the peptide may have a desired length. Preferably each branch is less than 24 amino acids long. Branching of the peptide may be effected by branching the peptide during synthesis on Lys residues by known methods. In this manner the peptide is branched on a first Lysine residue into two branches and further branched on further lysine residues to form a tetravalent entity thereafter. Other valencies, such as octamers, may be effected by including more or less branching steps. Odd valencies are also achievable by only partially branching the synthetic peptide.

Overall the chemical synthesis of the dendimer as the multivalent entity will allow controlling the nature and stoichiometry of oligomer very precisely. This in turn translates in higher precision in the various uses of the oligomer such as in T-cell labelling or detection.

Fig. 3 shows a branched peptide or dendrimer suitable for multimerising MHC complex monomers at their peptide according to the invention. The figure illustrates a polypeptide backbone that is branched twice to yield a tetrameric structure. The dendrimer is synthesized to incorporate the complementary recognition site (H') on each terminal branch, which can bind to the specific attachment site (H) under conditions that do not affect the stability of the MHC complexes, thus yielding a tetravalent entity. The figure further shows a fluorescent labelling moiety (F), which is incorporated in the unbranched portion of the dendrimer.

In an alternate embodiment the multivalent entity is an oligonucleotide or a cyclic oligonucleotide. The oligonucleotide will typically be 50 to 150 nucleic acids in length, but it may be as short as 3 nucleic acids. Preferably it is a cyclic oligonucleotide, which even more preferably is of less than 150 bases in length of circumference. This alternative embodiment is illustrated in Fig. 4, which shows the backbone of such a cyclic oligonucleotide. In analogy to Fig 3, here the oligonucleotide has been modified to include complementary recognition sites (H') on six different nucleotides, either during or after its synthesis, thus yielding a hexavalent entity. In addition the cyclic oligonucleotide also includes two fluorescent labelling moieties (F).

According to the second alternative for oligomerisation the MHC oligomer is through alignment of the peptides in their oligomerisation domains. With alignment we mean in the broadest sense any self-organized binding phenomenon between (macro)molecules, which result in at least a temporary association of two or more such (macro)molecules. Examples of this type are the hybridization of nucleic acids, the self assembly of two or more proteins (or protein domains) such as immunoglobulin Fc domains, coiled coil domains; self assembly of keratin fibres and similar and the covalent bonding of chemical moieties. The oligomerisation domains may actually form part of the peptidic backbone or may be attached thereto. They may be of peptide or nucleic acid character. Accordingly, in a first embodiment each peptide comprises a peptidic oligomerisation domain, which is preferably fused to the peptide backbone. In an alternative embodiment each peptide has attached thereto an oligomerisation domain. In this case the oligomerisation domain may be selected from the group consisting of a peptidic oligomerisation domain and a nucleic acid.

The peptidic oligomerisation domain may be selected from the group consisting of an antibody constant domain, an enzyme monomer and an oligomerisation domain of an oligomer-forming coiled-coil protein. Preferably, it is an oligomerisation domain of an oligomer-forming coiled-coil protein selected from the group consisting of the protein families of collagens, C-type lectins and thrombospondin family proteins.

Examples for oligomer-forming coiled-coil proteins include various types of collagen, triple coiled-coil domains of C-type lectins, such as mannose binding protein (MBP); Clq, myosin, leucine zippers such those occurring in p53, GCN4, bacteriophage P22 Mnt repressor; and the trombospondin family proteins such as COMP. Preferably the oligomerisation domain is derived from the cartilage oligomeric matrix protein (COMP). More preferably, the oligomerisation domain is of the human version of COMP.

The number of MHC complexes (m) comprised on the MHC oligomer of the invention will in the case of coiled coil proteins typically depend on the type of oligomerisation domain of the peptide is derived from and can in general be 2 or more, preferably m = 2 to 10, most preferably m = 3 or 5. If the modification of the peptides to be oligomerized is e.g. derived from the pentamerisation domain of the COMP this number will typically be five such that the oligomer will be a pentamer (m = 5), whereas in case these oligomerisation domains are derived from collagen this number will be three (m = 3).

In one embodiment each peptide sequentially comprises two non-overlapping oligomerisation domains. The second oligomerisation domain of the n^{th} peptide in the oligomer then dimerises or aligns with the first oligomerisation domain of the (n+1)^{th} peptide in the oligomer. The second oligomerisation domain of the (n + 1)^{th} peptide in the oligomer then dimerises or aligns with the first oligomerisation domain of the (n+2)^{th} peptide in the oligomer and so on. Most preferably in this case, the second oligomerisation domain of the last peptide in the oligomer dimerises with the first oligomerisation domain of the first peptide in the oligomer to provide a cyclic structure. Although for this embodiment in principle any type of oligomerisation domain capable of dimerisation can be used, the first and second oligomerisation domains are preferably nucleic acids, oligomerisation occurring by hybridization thereof.

The functional monomeric MHC complexes to be oligomerised will usually be soluble isolated or recombinant MHC complexes that may be derived from MHC class I or class II complexes, preferably the extra-cellular part of an MHC class I complex or the extra-cellular part of an MHC class II complex as shown in Fig. 1 or Fig. 2, respectively. Each of these complexes consists of an alpha chain and a beta chain. The functional complex further comprises the modified peptide bound in the respective groove formed by its alpha and beta chains.

The MHC proteins may be from any vertebrate species, e.g. primate species, particularly humans; rodents, including mice, rats, hamsters, and rabbits; equines, bovines, canines, felines; etc. Of particular interest are the human HLA proteins, and the murine H-2 proteins. Included in the HLA proteins are the class II subunits HLA-DPα, HLA-DPβ, HLA-DQα, HLA-DQβ, HLA-DRα and HLA-DRβ, and the class I proteins HLA-A, HLA-B, HLA-C, and β2 microglobulin. Included in the murine H-2 subunits are the class I H-2K, H-2D, H-2L, and the class II I-Aα, I-Aβ, I-Eα and I-Eβ, and microglobulin. Amino acid sequences of some representative MHC proteins are referenced in EP 812 331. Also included in the scope of this invention are non-classical examples such as HLA-E, HLA-F, HLA-G, Qa1, and CD1. The CD1 monomer may instead of the peptide have a lipid bound in its groove. The present invention is also applicable to the situation where a lipid instead of a peptide is bound and the skilled worker will be capable of translating the above oligomerisation protocols to this situation.

In a preferred embodiment, the MHC peptide chains correspond to the soluble form of the normally membrane-bound protein. For class I subunits, the soluble form is derived from the native form by deletion of the transmembrane and cytoplasmic domains. For class I proteins, the soluble form will include the α1, α2 and α3 domains of the α chain. For class II proteins the soluble form will include the α1 and α2 or β1 and β2 domains of the α chain or β chain, respectively.

Not more than about 10, usually not more than about 5, preferably none of the amino acids of the transmembrane domain will be included. The deletion may extend as much as about 10 amino acids into the α3 domain. Preferably none of the amino acids of the α3 domain will be deleted. The deletion will be such that it does not interfere with the ability of the α3 domain to fold into a functional disulfide bonded structure. The class I β chain, β2m, lacks a transmembrane domain in its native form, and does not have to be truncated. Generally, no class II subunits will be used in conjunction with class I subunits.

The above deletion is likewise applicable to class II subunits. It may extend as much as about 10 amino acids into the α2 or β2 domain, preferably none of the amino acids of the α2 or β2 domain will be deleted. The deletion will be such that it does not interfere with the ability of the α2 or β2 domain to fold into a functional disulfide bonded structure.

One may wish to introduce a small number of amino acids at the polypeptide termini, usually not more than 25, more usually not more than 20. The deletion or insertion of amino acids will usually be as a result of the requirements in cloning, e.g. as a consequence of providing for convenient restriction sites or the like, and to manage potential steric problems in the assembly of the molecules. In addition, one may wish to substitute one or more amino acids with a different amino acid for similar reasons, usually not substituting more than about five amino acids in any one domain.

In general, the oligomer may thus in one or more of the proteins or peptide chains comprised therein further comprise one or more additional domains such as one or more linkers, a tagging domain and a purification domain. The additional domain(s) may e.g. be provided on the multivalent entity or the peptide, but may also be present on the MHC alpha and/or beta chains.

For illustration purposes, the complexes in Fig. 1 and Fig. 2 additionally have a protein tag (E) fused to one of their protein chains as a fusion polypeptide, which tag may allow further labelling, purification or attachment of these proteins. The tag E may be an epitope tag, or otherwise modified protein sequence. This tag may be separated from the polypeptide of the MHC alpha or beta chain by a polypeptide linker. In general this and any other linker will comprise not more than 25, preferably not more than 20 amino acids.

The protein tag (E) optionally included in the complexes as described above can be any domain wich allows for labeling of the protein. Preferably the tagging domain includes a label. This label can be included in the domain itself such as an epitope recognised by an antibody or a light detectable or radioactive label. Preferably, the label is selected from the group consisting of fluorescent markers, such as such as FITC, phycobiliproteins, such as R- or B-phycoerythrin, allophycocyanin, Cy3, Cy5, Cy7, a luminescent marker, a radioactive label such as ¹²⁵I or ³²P, an enzyme such as horseradish peroxidase, or alkaline phosphatase e.g. alkaline shrimp phosphatase, an epitope, a lectin, biotin or streptavidin.

Where the label is itself a protein, the polypeptide chain of the protein used for labeling can be fused to the peptide to form a chimeric protein, preferably at its C terminus. For example a fluorescent protein such as a green-fluorescent protein (GFP), or a subunit of a phycobiliprotein could be used in this chimeric protein. GFP chimeric protein technology is well known in the art. Chimeric proteins comprising a suitable domain from a phycobiliprotein is described, for example in WO 01/46395.

Alternatively labeling can be achieved by binding of a suitably labeled antibody, or antibody fragment, such as a labeled F(ab) fragment to a suitable epitope on the tag (E).

The purification domain optionally to be included in the oligomers of the invention can be any domain assisting in purification of the protein of the invention e.g. by providing specific binding characteristics. Appropriate sequences are known to the skilled worker and can be applied as long as they do not interfere with the functional parts of the complexes. Preferably the purification domain is a hexahistidine sequence.

The functional monomeric MHC complexes to be oligomerised comprise the peptide bound in their peptide binding groove. This peptide comprises at least the MHC binding portion and a separate portion forming or bearing the modification in form of an attachment site or oligomerisation domain. The MHC binding portion, excluding any linker and the modification, will be from about 6 to 14 amino acids in length for complexes with class I MHC proteins, and usually about 8 to 11 amino acids. It will be from about 6 to 35 amino acids in length for complexes with class II MHC proteins, usually from about 10 to 20 amino acids.

The peptides may have in their MHC binding portion a sequence derived from a wide variety of proteins. In many cases it will be desirable to use peptides, which act as T cell epitopes. The epitope sequences from a number of antigens are known in the art. Alternatively, the epitope sequence may be empirically determined by isolating and sequencing peptides bound to native MHC proteins, by synthesis of a series of putative antigenic peptides from the target sequence, then assaying for T cell reactivity to the different peptides, or by producing a series of MHC-peptide complexes with these peptides and quantification the T cell binding. Preparation of peptides, including synthetic peptide synthesis, identifying sequences, and identifying relevant minimal antigenic sequences is known in the art. In any case, the peptide comprised in the oligomeric MHC complex is preferably substantially homogeneous, meaning that preferably at least 80%, more preferably at least 90 % and most preferably at least 95% of the peptides are identical with regard to their MHC binding portion.

Typically a linker polypeptide sequence will be interposed between the sequence of the MHC binding portion of the peptide (P) and the site of modification (H). Such linker may e.g. be a repeat of glycine residues, interspersed with prolines or serines, for flexibility and solubility, (GGPGG)ₙ or (GGSGG)ₙ with n typically ranging between 1 and 6. It will be appreciated that other linkers, which are flexible, have sufficient solubility and do not form significant secondary structure will also be suitable for this purpose. In general, polypeptide linkers of 1 to 30, preferably 3 to 20 and most preferably 3 to 10 amino acids in length may be used. For non-peptidic linkers their length may be adjusted accordingly. Non-peptidic linkers can also include PEG or poly ethylene oxide (PEO) repeats.

Instead of providing the linker between the sequence of the MHC binding portion of the peptide (P) and the site of modification (H) by solid phase synthesis, this linker can be inserted by chemoselective ligation as described herein.

In one embodiment the MHC binding portion of the peptide is synthesized with a short N-termnial linker GGSGG which itself carries an N-termnial cysteine. MHC-peptide monomers with this peptide are formed as described elsewhere herein. After formation and publication of the monomers a second longer linker segment is coupled to the cysteine, with the structure R₁-R₂-R₃, wherein R₁ is a moiety that selectively binds to N-terminal cysteines, such as thioester, aldehyde, or maleimide, R₂ is an amino acid or PEG or PEO or other polymer repeat linker and R₃ is a second ligation moiety such as biotin or another moiety as described herein. In the case where R₁ is maleimide, the cysteine can also be appended via a similar linker as a C terminal amino acid to the C-terminus of the MHC binding portion of the peptide. The monomers with the additional longer linker R₂ can then be coupled to the multivalent entity to form the desired multimers via R₃. This has the advantage that the synthesis of very long peptides including the MHC binding portion can be alleviated. In the case of R₃ being biotin the multivalent entity will be an avidin-family protein, such as streptavidin or a cross-linked version thereof in order to increase the number of accessible binding sites and therefore the valency of the resulting multimeric binding complex.

The length of the linker is preferably chosen so that the oligomerisation domain or attachment site is at least far enough removed from the location of binding of the MHC-peptide complex to the T cell receptor (TCR) and the CD4, CD8 or other co-receptors such that it does not substantially interfere with the respective interaction of the MHC-peptide complex with the TCR. Subject to the foregoing objective, the linker length will also be kept as short as possible, as this will help to reduce the cost of peptide synthesis.

MHC oligomer according to the invention may also comprise a label. Such label may for example be selected from the group consisting of a light detectable label, a radioactive label, an enzyme, an epitope, a lectin, or biotin. Other labels have been listed above with regard to the tagging domain E. These may be used as well. If such entity is present, the label is in one embodiment preferably provided on the multivalent entity or structure formed by alignment of the oligomerisation domains. The label may be incorporated during or after assembly of the oligomer or even during application or use of the same. Where the multivalent entity itself is formed by chemical synthesis the label may be chemically introduced at a predetermined location on the multivalent entity which will allow labeling of the oligomeric MHC complexes with precise stoichiometry. Where the label is a fluorescent label this will allow obtaining complexes with high uniform brightness and maxima signal to noise ratio.

The present invention also pertains to a pharmaceutical or diagnostic composition comprising an MHC oligomer as defined above, optionally in combination with a pharmaceutically acceptable carrier.

Pharmaceutical compositions comprising the oligomers of the invention are useful for, e.g. parenteral administration, i.e. subcutaneously, intramuscularly or intravenously. In addition, a number of new drug delivery approaches are being developed. The pharmaceutical compositions of the present invention are suitable for administration using these new methods, as well.

The compositions for parenteral administration will commonly comprise a solution of the oligomer dissolved in an acceptable carrier, preferably an aqueous carrier. A variety of aqueous carriers can be used, e.g. buffered water, 0.4 % saline, 0.3 % glycine and the like. These solutions are sterile and generally free of particulate matter. These compositions may be sterilized by conventional, well-known sterilization techniques. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. The concentration of the oligomer in these formulations can vary widely, i.e. from less than about 1 pg/ml, usually at least about 0.1 mg/ml to as much as 10 - 100 mg/ml and will be selected primarily based on fluid volumes, viscosities, etc. in accordance with the particular mode of administration selected.

A typical pharmaceutical composition for intramuscular injection could be made up to contain 1 ml sterile buffered water, and 0.1 mg of oligomer protein. A typical composition for intravenous infusion could be made up to contain 250 ml of sterile Ringer's solution, and 10 mg of oligomer complex protein. Actual methods for preparing parenterally administrable compositions will be known or apparent to those skilled in the art.

The MHC oligomers of this invention can be lyophilized for storage and reconstituted in a suitable carrier prior to use. This technique has been shown to be effective and commonly used lyophilization and reconstitution techniques can be employed. It will be appreciated by those skilled in the art that lyophilization and reconstitution can lead to varying degrees of activity loss and that use levels may have to be adjusted to compensate.

The present invention also relates to a method of labeling and/or detecting mammalian T cells according to the specificity of their antigen receptor, the method comprising
(i) combining an MHC oligomer according to the invention and a suspension or biological sample comprising T cells, and
(ii) detecting the presence of specific binding of said complex and the T cells.

The present invention also relates to a method of separating mammalian T cells according to the specificity of their antigen receptor, the method comprising
(i) combining an MHC oligomer according to the invention and a suspension or biological sample comprising T cells, and
(ii) separating T cells bound to said complex from unbound cells.

Further the invention also relates to a method for labeling and/or detecting and/or separating cells as described above wherein instead of T cells a type of lymphocyte is labeled, detected or separated according to a specific type of cell surface molecule that specifically binds to an MHC molecule. For example it is known that the human non-classical MHC molecule HLA-E can bind to Natural Killer (NK) cells via specific binding to the NK surface receptor CD94/NKG2 (D.S.J. Allan et al., J. Immunol. Meth. 268 (2002) 43-50). Similarly HLA-G and HLA-F was shown to stain ILT2 and ILT4 receptors on CD14+ cells and HLA-F was also shown to stain a sub-population of CD19+ (Allan *et al., supra*)*.* The skilled worker will be capable of translating the methods described above to this situation..

The present invention finally relates to a method of forming an MHC oligomer comprising at least two functional MHC complexes having a peptide binding groove, each MHC complex having a peptide bound in the peptide binding groove of the MHC complex, wherein each peptide comprises a modification allowing for highly specific oligomerisation, said method comprising the steps of:
(i) providing one or more peptides capable of binding in the peptide groove of each functional MHC complex, said peptide being modified by
   (a) providing a specific attachment site, or
   (b) providing an oligomerisation domain;
(ii) providing monomeric functional MHC-peptide complexes including the peptide of (i),
   and
(iii) oligomerising the functional MHC complexes through
   (a) providing a multivalent entity and binding of the said monomeric functional MHC-peptide complexes to the multivalent entity at the specific attachment site provided on or attached to each peptide, or
   (b) alignment of the peptides through the oligomerisation domain provided on or attached to each peptide.

Preferably, the monomeric functional MHC complexes are provided in step (ii) by refolding the MHC α and β chains in presence of the peptide as modified in step (i).

The MHC α and β chains may e.g. be obtained from an eukaryotic or prokaryotic expression system and may optionally purified before refolding. Alternatively, the α and β chains or the folded complexes may be isolated from other sources. The method may, where desired or appropriate also comprise the step of peptide exchange for refolded functional MHC complexes. Providing the peptide in step (i) will typically occur by solid phase synthesis as disclosed above. It may be modified either during or after said synthesis. Modification during synthesis will e.g. be by adding amino acids of peptide domains to the backbone or by incorporation of modified (e.g. thio-) amino acids. Modification after synthesis may e.g. be by attachment of a nucleic acid oligomerisation domain.

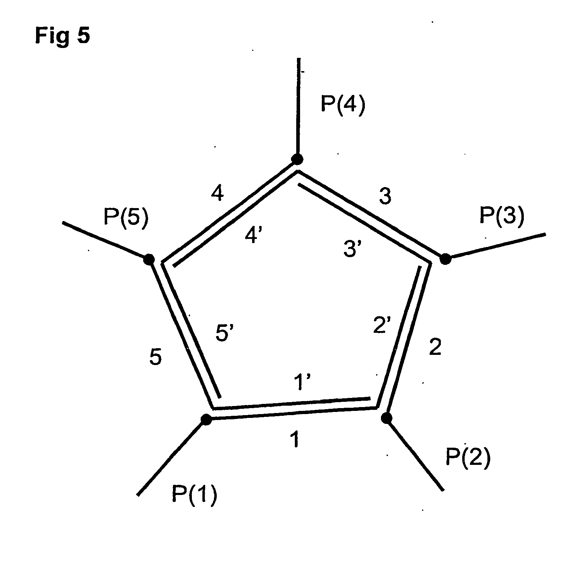

In Fig. 5 modified peptides (P) are shown that enable forming multimeric MHC-peptide complexes without a separate multivalent entity. The antigenic peptide will typically have a 10 amino acid glycine serine linker to provide sufficient spacing in the oligomer. The peptide is synthesized in five variants P(1) to P(5) to comprise five different oligonucleotides. Preferably P(1) to P(5) will comprise the same MHC binding portion.

Each attached oligonucleotide has two domains (1,2') (2,3'), (3,4'), (4,5'), and (5,1'), of which each one is complementary to one and only one other domain on another oligonucleotide. The fifth oligonucleotide has in a second domain 1' that is complementary to the first domain 1 of the first oligonucleotide. Each domain pair may e.g. have an annealing temperature of about 20°C. The cross-reactivity between non-complementary domains is to be minimized by known techniques of oligonucleotide design. The two domains on each oligonucleotide may be-directly connected or have a non-annealing oligonucleotide linker, increasing the spacing between the domains. The linker may comprise a synthetic label, such as the labels described herein before. The coupled polypeptide-oligonucleotide may be synthesized in a single process or be synthesized separately with coupling carried out afterwards, wherein the peptide to oligonucleotide linkage can be achieved through chemoselective ligation of modified peptide to oligonucleotide as described above. The five oligo/peptide products are each purified to sufficient purity by HPLC.

The oligo/peptides are then annealed at 20 °C after heating to 30 °C for 5min. Oligo/peptide pentamers are then re-purified. Oligo/peptide pentamers are then refolded with denatured MHC alpha and beta chain according to known protocols. This will result in hetero-multimers of non-uniform valency being formed, as the refolding of MHC molecules is imperfect and given a suitable peptide excess it will mainly result in MHC-peptide monomers which have 4 additional peptides attached via oligo coupling (MHC-P₅). The resulting product is then concentrated and purified by gel-filtration chromatography to predominantly select MHC-P₅ monomers. The P5 interaction is then melted by heating the monomers to 30°C for 5 minutes, resulting in MHC-P' monomers and free peptide. The peptide is then purified away rapidly on a PD10 column (Amersham Biosciences, Chalfont St. Giles, UK) at 30°C. The resulting MHC-P monomers are then eluted from the column. As the individual P(1) to P(5) peptides will occur with roughly equal molar ratio in the purified product the individual MHC-P monomers can then be reannealed to form MHC pentamers. Incorrectly annealed products are purified away in a second gel filtration step so that the purified product substantially only includes MHC pentamer.

### Examples:

### Example 1: Formation of a labelled class II MXC peptide octamer HLA-DRB1*0101 incorporating the Influenza Hemagglutinin A peptide HA (306-318): PKYVKQNTLKLAT

HA'-peptide is synthesized as HA-(GGGSG)₂-K" is synthesized whereby K" is Lysine modified to include benzaldehyde to > 95% purity.

Generally a peptide P'-K"-P" where P', P" are two polypeptide sub-segments can be synthesized as follows. An Fmoc-Lys(Mtt)-OH or Fmoc-Lys(ivDde)-OH residue is introduced as the position that is desired for the modification, wherein Fmoc is 9-fluorenyl methoxycarbonyl, Mtt is 4-methyltrityl, and ivDde is 1-(4,4-dimethyl-2,6-dioxocyclohex-1-ylidene)-3-methylbutyl. The N-terminal amine of the peptide is then blocked with butoxycarbonyl (Boc) so that the Mtt or ivDde can be selectively removed. The exposed amine groups can then be modified either with acetone 5-(succinimidyloxycarbonyl)-pyridine-2-ylhydrazone (SANH) or succinimidyl-4-formylbenzoate (SFB) to introduce either a 2-hydrazinopyridyl or a benzaldehyde group.

Monomeric HLA-DRB1*0101 HA' complexes are refolded from inclusion body material of the soluble alpha chain HLA-DRA1*0101 and beta chain HLA-DRB1*0101 and HA' peptide following the protocol as described in Cameron et al., J. Immunol. Meth. 268: (2002) 51-69 with the difference that no fusion tag for biotinylation is required on either protein chain. Purification of the monomeric complexes is achieved as described in the same protocol. The complexes are assessed for protein concentration by the method of Bradford.

The following octavalent peptide (F)MV8 is synthesized.

(F)MV8.1: (K'''GGSGGGGSGG)₈(K'G)₄(K'G)₂K'GK^{F}GA

Branching of this peptide occurs at each lysine K' and the numerals indicated the number of branches in the peptide at each stage. K^{F} is a Lysine residue modified with fluorescein isothiocyanate FITC to provide a fluorescent marker. K"' is a Lysine residue modified with 2-hydrazinopyridyl, providing eight 2-hydrazinopyridyl groups on the branched peptide that can react with the aldehyde group on the HA' peptide to form stable bis-aromatic hydrazone.

Momomeric HLA-DRB1*0101 HA' are mixed with (F)MV8.1 at a 8:1 molar ratio in PBS at pH6.8 to form FITC labelled HLA-DRB1*0101 HA' octamers.

The resulting product is purified by gel filtration chromatography on a Sephadex S 300 column (Amersham Biosciences, Chalfont St.Giles, UK) to recover predominantly the octameric species.

### Example2: Formation of a labelled class I MHC peptide octamers HLA-A *0201 incorporating the CMV pp65 peptide (495-503): NLVPMVATV:

The peptide NLV':NLVPMVATV-(GGGSG)₂-K" is synthesized to > 95% purity, where K" is as described above.

Monomeric HLA-A*0201 / NLV' complexes are refolded from inclusion body material of the soluble alpha chain HLA-A*0201 and beta 2 microglobulin and the NLV' peptide following the protocol as described in Garboci et al., PNAS 89 (1992), 3429-3433. Purification of the monomeric complexes is achieved as described in the same protocol. The complexes are assessed for protein concentration by the method of Bradford. (F)MV8.2: A cyclic oligo of 96 bases is synthesized according to preferred methods as described hereinbefore, wherein every twelfth base is modified with 2-hydrazinopyridyl and each base in the centre between two 2-hydrazinopyridyl moieties is modified to include (FITC) according to methods standard oligonucleotide synthesis. The choice of bases will be such that they are convenient for modification and minimize secondary structure.

Monomeric HLA-A*0201 NLV' complexes are mixed with (F)MV8.2 at a 8:1 molar ratio in reaction buffer at pH6.8 to form FITC labelled HLA-A*0201 NLV' octamers.

The resulting product is purified by gel filtration chromatography, e.g. on a Sephadex S 300 column (Amersham Biosciences, Chalfont St.Giles, UK) to recover predominantly the octameric species.

In each example above the resulting product is purified by gel filtration chromatography, on a Sephacryl S-300 column (Amersham Biosciences, Chalfont St.Giles, UK) to recover predominantly the octameric species. The purified product may be concentrated but otherwise it is ready for use, e.g. in flow cytometry.

It will be understood that either multivalent entity (F)MV8.1 or (F)MV8.2 can be used interchangeably to multimerise class I and class II MHC complexes, respectively.

## Claims

1. MHC oligomer comprising at least two functional MHC complexes having a peptide binding groove, each MHC complex having a peptide bound in the peptide binding groove of the MHC complex,
wherein each peptide has a modification which allows, in a predetermined manner, specific and/or selective formation of an oligomer of the functional MHC complexes through a core structure, said core structure being an entity allowing a simultaneous binding of each peptide.

2. The MHC oligomers of claim 1, wherein the MHC complexes have been oligomerised at their peptides after assembly of the functional monomeric MHC complexes including the peptide.

3. The MHC oligomers of claim 1 or 2, wherein each peptide comprises a modification selected from the group consisting of a specific attachment site and an oligomerisation domain,
wherein oligomerisation of the functional MHC complexes occurs through
(i) binding of each peptide to a multivalent entity at the specific attachment site provided on or attached to each peptide, or
(ii) alignment of the peptides through the oligomerisation domain provided on or attached to each peptide
wherein the MHC complexes in the oligomer are connected to a core structure provided by the multivalent entity or by alignment of the peptides.

4. MHC oligomer of claim 3, wherein oligomerisation occurs through binding of the peptide to a multivalent entity.

5. MHC oligomer of claim 4, wherein the binding occurs through recognition of a specific attachment site provided on the peptide by a recognition site provided on the multivalent entity.

6. MHC oligomer of any one of claims 3 to 5, wherein the peptide comprises an MHC binding portion and a separate portion bearing or forming the modification.

7. MHC oligomer of claim 6, wherein the peptide further comprises a polypeptide linker between its MHC binding portion and the separate portion, the linker being preferably 3-10 amino acids in length.

8. MHC oligomer of claim 5, wherein the specific attachment site and the recognition site are selected from members of a specific binding pair.

9. MHC oligomer of claim 8, wherein the specific binding pair is selected from the group consisting of hapten/antibody, epitope/antibody, ligand/receptor, substrate or substrate analogon/enzyme, cofactor or cofactor analogon/enzyme, nucleic acid/complementary nucleic acid, sugar/lectin, biotin/avidin family protein, such as avidin, streptavidin, neutravidin, Streptag® / Streptactin®.

10. MHC oligomer of anyone of claims 4 to 7, wherein the attachment occurs through covalent binding of the peptide to the multivalent entity and the attachment site and the recognition site are moieties capable of creating a covalent bond with each other.

11. MHC oligomer of claim 10, wherein the covalent bond formed is of a type selected from the group consisting of oxime, hydrazone, thiazolidine, oxazolidine, thioether, disulfide, and peptide.

12. MHC oligomer of anyone of claims 4 to 11, wherein the multivalent entity is selected from the group consisting of a natural polymer or derivative thereof such as a protein, a branched polypeptide (dendrimer), a multimeric protein, a nucleic acid, a polysaccharide, such as dextran, starch, cellulose, hyaluronic acid, chitin, or alginic acid or a derivative of these polysaccharides, an oligonucleotide, a cyclic oligonucleotide; a synthetic polymer; a phospholipid membrane such as a vesicle or a liposome; and an inorganic particle.

13. MHC oligomer of claim 12, wherein the multivalent entity is a natural or synthetic polymer and the recognition sites are provided by the backbone of the multivalent entity, or are attached thereto.

14. MHC oligomer of claim 11, wherein the multivalent entity is a branched polypeptide (dendrimer), preferably having a recognition site incorporated at predetermined sites in two or more of its branches.

15. MHC oligomer of claim 14, wherein each branch of the peptide is less than 24 amino acids long.

16. MHC oligomer of claim 10, wherein the multivalent entity is an oligonucleotide or a cyclic oligonucleotide, the cyclic oligonucleotide preferably being of less than 150 bases in length of circumference.

17. MHC oligomer of claim 3, wherein oligomerisation occurs through alignment of the peptides.

18. MHC oligomer of claim 17, wherein each peptide has attached thereto an oligomerisation domain preferably selected from the group consisting of a peptidic oligomerisation domain and a nucleic acid.

19. MHC oligomer of claim 18 wherein the oligomerisation domain is a peptidic oligomerisation domain, which is selected from the group consisting of an antibody constant domain, keratin and an oligomerisation domain of an oligomer-forming coiled-coil protein.

20. MHC oligomer of claim 18, wherein each peptide sequentially comprises two non-overlapping oligomerisation domains, and wherein the second oligomerisation domain of the n^{th} peptide in the oligomer dimerises with the first oligomerisation domain of the (n+1)^{th} peptide in the oligomer.

21. MHC oligomer of claim 20, wherein further the second oligomerisation domain of the last peptide in the oligomer dimerises with the first oligomerisation domain of the first peptide in the oligomer to provide a cyclic structure.

22. MHC oligomer of claim 20 or 21, wherein the first and second oligomerisation domains are nucleic acids.

23. MHC oligomer of one of the preceding claims, wherein the MHC oligomer substantially does not contain sections of MHC-binding peptide that are not bound to an MHC-peptide complex.

24. MHC oligomer of one of the preceding claims, wherein the MHC complex is derived from the extra-cellular part of an MHC class I complex.

25. MHC oligomers of any one of the preceding claims wherein the MHC complex is derived from the extra-cellular part of an MHC class II complex.

26. MHC oligomers of any one of the preceding claims wherein the peptides are substantially homogeneous in their MHC binding portion.

27. MHC oligomer according to any of the preceding claims, wherein at least one of the monomeric functional MHC complexes is CD1 and the corresponding peptide is a lipid.

28. MHC oligomer of one of the preceding claims, wherein substantially none of the amino-acid side chains of the MHC binding portion of the peptide and/or of the MHC alpha or beta chains in the MHC-peptide complexes comprised in the oligomer have been modified in the process of oligomerisation.

29. Pharmaceutical or diagnostic composition comprising an MHC oligomer according to any one of the preceding claims, optionally in combination with a pharmaceutically acceptable carrier.

30. Method of labeling and/or detecting mammalian T cells according to the specificity of their antigen receptor, the method comprising
(i) combining an MHC oligomer according to any one of claims 1 to 28 and a suspension or biological sample comprising T cells, and
(ii) detecting the presence of specific binding of said complex and the T cells.

31. Method of separating mammalian T cells according to the specificity of their antigen receptor, the method comprising
(i) combining an MHC oligomer according to any one of claims 1 to 28 and a suspension or biological sample comprising T cells, and
(ii) separating T cells bound to said complex from unbound cells.

32. The method of claim 30 or claim 31 above wherein instead of T cells a type of lymphocyte is labeled, detected or separated according to a specific type of cell surface molecule that specifically binds to an MHC molecule.

33. Method of forming an MHC oligomer as defined in one of claims 1 to 28 and comprising at least two functional MHC complexes having a peptide binding groove, each MHC complex having a peptide bound in the peptide binding groove of the MHC complex, wherein each peptide has a modification which allows highly specific oligomerisation of the functional MHC complexes through a core structure, said method comprising the steps of:
(i) providing one or more peptides capable of binding in the peptide groove of each functional MHC complex, said peptide being modified by
(a) providing a specific attachment site, or
(b) providing an oligomerisation domain;
(ii) providing monomeric functional MHC-peptide complexes including the peptide modified in (i),
and
(iii) oligomerising the functional MHC complexes through
(a) providing a multivalent entity and binding of the said monomeric functional MHC-peptide complexes to the multivalent entity at the specific attachment site provided on or attached to each peptide, or
(b) alignment of the peptides through the oligomerisation domain provided on each peptide or attached to each peptide.

34. The method of claim 33, wherein the monomeric functional MHC-peptide complexes are provided in step (ii) by refolding the MHC α and β chains in presence of the peptide as modified in step (i).

35. The method of claim 33, wherein the monomeric functional MHC-peptide complexes are provided in step (ii) by peptide exchange for refolded functional MHC complexes in the presence of the peptide as modified in step (i).

## Patentansprüche

1. MHC-Oligomer umfassend mindestens zwei funktionelle MHC-Komplexe, die eine Peptid-bindende Spalte aufweisen, wobei jeder MHC-Komplex ein Peptid in der Peptid-bindenden Spalte des MHC-Komplexes gebunden hat,
wobei jedes Peptid eine Modifikation aufweist, die in einer vorgegebenen Weise die spezifische und/oder selektive Bildung eines Oligomers der funktionellen MHC-Komplexe über eine Kernstruktur erlaubt, wobei die Kernstruktur eine Einheit ist, die eine gleichzeitige Bindung von jedem Peptid erlaubt.

2. MHC-Oligomere gemäß Anspruch 1, wobei die MHC-Komplexe an deren Peptiden nach der Zusammenfügung der funktionellen monomeren MHC-Komplexe einschließlich des Peptids oligomerisiert wurden.

3. MHC-Oligomere gemäß Anspruch 1 oder 2, wobei jedes Peptid eine Modifikation umfasst, ausgewählt aus der Gruppe bestehend aus einer spezifischen Bindungsstelle und einer Oligomerisierungsdomäne, wobei Oligomerisierung der funktionellen MHC-Komplexe durch
(i) Bindung jedes Peptids an eine multivalente Einheit an der spezifischen Bindungsstelle, die an jedem Peptid bereitgestellt oder daran befestigt ist, oder
(ii) Ausrichtung der Peptide durch die Oligomerisierungsdomäne, die an jedem Peptid bereitgestellt oder daran befestigt ist,
erfolgt, wobei die MHC-Komplexe in dem Oligomer an eine Kernstruktur gekoppelt sind, die durch die multivalente Einheit oder die Ausrichtung der Peptide bereitgestellt ist.

4. MHC-Oligomer gemäß Anspruch 3, wobei Oligomerisierung durch Bindung des Peptids an eine multivalente Einheit erfolgt.

5. MHC-Oligomer gemäß Anspruch 4, wobei die Bindung durch Erkennung einer spezifischen Bindungsstelle erfolgt, die an dem Peptid durch eine Erkennungsstelle bereitgestellt ist, wobei die Erkennungsstelle an der multivalenten Einheit bereitgestellt ist.

6. MHC-Oligomer gemäß einem der Ansprüche 3 bis 5, wobei das Peptid einen MHC-Bindeteil und einen separaten Teil umfasst, der die Modifikation enthält oder bildet.

7. MHC-Oligomer gemäß Anspruch 6, wobei das Peptid weiter einen Polypeptidlinker zwischen seinem MHC-Bindeteil und dem separaten Teil umfasst, wobei der Linker vorzugsweise 3 bis 10 Aminosäuren lang ist.

8. MHC-Oligomer gemäß Anspruch 5, wobei die spezifische Bindungsstelle und die Erkennungsstelle ausgewählt sind aus Mitgliedern eines spezifischen Bindungspaars.

9. MHC-Oligomer gemäß Anspruch 8, wobei das spezifische Bindungspaar ausgewählt ist aus der Gruppe bestehend aus Hapten/Antikörper, Epitop/Antikörper, Ligand/Rezeptor, Substrat oder Substratanalogon/Enzym, Cofaktor oder Cofaktoranalogon/Enzym, Nucleinsäure/komplementäre Nucleinsäure, Zucker/Lektin, Biotin/Avidinfamilieprotein, wie Avidin, Streptavidin, Neutravidin, Streptag®/Streptactin®.

10. MHC-Oligomer gemäß einem der Ansprüche 4 bis 7, wobei die Bindung durch kovalente Bindung des Peptids an die multivalente Einheit erfolgt und die Bindestelle und die Erkennungsstelle Gruppen sind, die in der Lage sind, eine kovalente Bindung miteinander herzustellen.

11. MHC-Oligomer gemäß Anspruch 10, wobei die gebildete kovalente Bindung von einem Typ ist ausgewählt aus der Gruppe bestehend aus Oxim, Hydrazon, Thiazolidin, Oxazolidin, Thioether, Disulfid und Peptid.

12. MHC-Oligomer gemäß einem der Ansprüche 4 bis 11, wobei die multivalente Einheit ausgewählt ist aus der Gruppe bestehend aus einem natürlichen Polymer oder einem Derivat davon, wie einem Protein, einem verzweigten Polypeptid (Dendrimer), einem multimeren Protein, einer Nucleinsäure, einem Polysaccharid, wie Dextran, Stärke, Cellulose, Hyaluronsäure, Chitin oder Alginsäure oder einem Derivat dieser Polysaccharide, einem Oligonucleotid, einem cyclischen Oligonucleotid, einem synthetischen Polymer, einer Phospholipidmembran, wie einem Vesikel oder einem Liposom, und einem anorganischen Partikel.

13. MHC-Oligomer gemäß Anspruch 12, wobei die multivalente Einheit ein natürliches oder synthetisches Polymer ist und die Erkennungsstellen durch das Rückgrat der multivalenten Einheit bereitgestellt sind oder daran befestigt sind.

14. MHC-Oligomer gemäß Anspruch 11, wobei die multivalente Einheit ein verzweigtes Polypeptid (Dendrimer) ist, das bevorzugt eine Erkennungsstelle hat, die an bestimmten Stellen in zwei oder mehreren seiner Verzweigungen eingebaut ist.

15. MHC-Oligomer gemäß Anspruch 14, wobei jede Verzweigung des Peptids weniger als 24 Aminosäuren lang ist.

16. MHC-Oligomer gemäß Anspruch 10, wobei die multivalente Einheit ein Oligonucleotid oder ein cyclisches Oligonucleotid ist, wobei das cyclische Oligonucleotid bevorzugt weniger als 150 Basen Umfangslänge hat.

17. MHC-Oligomer gemäß Anspruch 3, wobei Oligomerisierung durch Ausrichtung der Peptide erfolgt.

18. MHC-Oligomer gemäß Anspruch 17, wobei an jedes Peptid eine Oligomerisierungsdomäne gebunden ist, bevorzugt ausgewählt aus der Gruppe bestehend aus einer peptidischen Oligomerisierungsdomäne und einer Nucleinsäure.

19. MHC-Oligomer gemäß Anspruch 18, wobei die Oligomerisierungsdomäne eine peptidische Oligomerisierungsdomäne ist, die ausgewählt ist aus der Gruppe bestehend aus einer konstanten Antikörperdomäne, Keratin und einer Oligomerisierungsdomäne eines Oligomer-bildenden Coiled-coil-Proteins.

20. MHC-Oligomer gemäß Anspruch 18, wobei jedes Peptid nacheinander zwei nicht überlappende Oligomerisierungsdomänen umfasst und wobei die zweite Oligomerisierungsdomäne des n-ten Peptids im Oligomer mit der ersten Oligomerisierungsdomäne des (n+1)-ten Peptids im Oligomer dimerisiert.

21. MHC-Oligomer gemäß Anspruch 20, wobei außerdem die zweite Oligomerisierungsdomäne des letzten Peptids im Oligomer mit der ersten Oligomerisierungsdomäne des ersten Peptids im Oligomer dimerisiert, um eine cyclische Struktur bereitzustellen.

22. MHC-Oligomer gemäß Anspruch 20 oder 21, wobei die erste und die zweite Oligomerisierungsdomäne Nucleinsäuren sind.

23. MHC-Oligomer gemäß einem der vorangehenden Ansprüche, wobei das MHC-Oligomer im Wesentlichen keine Abschnitte des MHC-bindenden Peptids enthält, die nicht an einen MHC-Peptid-Komplex gebunden sind.

24. MHC-Oligomer gemäß einem der vorangehenden Ansprüche, wobei der MHC-Komplex abgeleitet ist vom extrazellulären Teil eines MHC-Klasse-I-Komplexes.

25. MHC-Oligomer gemäß einem der vorangehenden Ansprüche, wobei der MHC-Komplex abgeleitet ist vom extrazellulären Teil eines MHC-Klasse-II-Komplexes.

26. MHC-Oligomer gemäß einem der vorangehenden Ansprüche, wobei die Peptide im Wesentlichen in deren MHC-Bindeteil homogen sind.

27. MHC-Oligomer gemäß einem der vorangehenden Ansprüche, wobei mindestens einer der monomeren funktionellen MHC-Komplexe CD1 ist und das entsprechende Peptid ein Lipid ist.

28. MHC-Oligomer gemäß einem der vorangehenden Ansprüche, wobei im Wesentlichen keine der Aminosäureseitenketten des MHC-Bindeteils des Peptids und/oder der MHC-alpha- oder beta-Ketten in den MHC-Peptidkomplexen, die im Oligomer enthalten sind, während des Oligomerisierungsprozesses modifiziert wurden.

29. Arzneimittel oder diagnostische Zusammensetzung umfassend ein MHC-Oligomer gemäß einem der vorangehenden Ansprüche, gegebenenfalls in Kombination mit einem pharmazeutisch verträglichen Träger.

30. Verfahren zum Markieren und/oder Nachweisen von Säuger T-Zellen gemäß der Spezifität ihres Antigenrezeptors, wobei das Verfahren umfasst:
(i) Kombinieren eines MHC-Oligomers gemäß einem der Ansprüche 1 bis 28 und einer Suspension oder biologischen Probe umfassend T-Zellen, und
(ii) Nachweisen der Anwesenheit einer spezifischen Bindung des Komplexes und der T-Zellen.

31. Verfahren zum Trennen von Säuger T-Zellen gemäß der Spezifität ihres Antigenrezeptors, wobei das Verfahren umfasst:
(i) Kombinieren eines MHC-Oligomers gemäß einem der Ansprüche 1 bis 28 und einer Suspension oder biologischen Probe umfassend T-Zellen, und
(ii) Trennen der T-Zellen, die an den Komplex gebunden sind, von ungebundenen Zellen.

32. Verfahren gemäß Anspruch 30 oder 31, wobei anstelle von T-Zellen ein Lymphocytentyp markiert, nachgewiesen oder getrennt wird gemäß einem spezifischen Zelloberflächenmolekültyp, der spezifisch an ein MHC-Molekül bindet.

33. Verfahren zur Bildung eines MHC-Oligomers wie in einem der Ansprüche 1 bis 28 definiert, wobei das MHC-Oliogmer mindestens zwei funktionelle MHC-Komplexe umfasst, die eine Peptid-bindende Spalte aufweisen, wobei jeder MHC-Komplex ein Peptid in der Peptid-bindenden Spalte des MHC-Komplexes gebunden hat,
wobei jedes Peptid eine Modifizierung aufweist, die eine hochspezifische Oligomerisierung der funktionellen MHC-Komplexe über eine Kernstruktur erlaubt, wobei das Verfahren die Schritte umfasst:
(i) Bereitstellen eines oder mehrerer Peptide, die in der Lage sind, in der Peptid-Spalte jedes funktionellen MHC-Komplexes zu binden, wobei das Peptid modifiziert ist durch
(a) Bereitstellen einer spezifischen Bindestelle; oder
(b) Bereitstellen einer Oligomerisierungsdomäne;
(ii) Bereitstellen monomerer funktioneller MHC-Peptidkomplexe, die das Peptid wie in (i) modifiziert beinhalten,
und
(iii) Oligomerisieren der funktionellen MHC-Komplexe durch
(a) Bereitstellen einer multivalenten Einheit und Binden der monomeren funktionellen MHC-Peptid-Komplexe an die multivalente Einheit an der spezifischen Bindestelle, die an jedem Peptid bereitgestellt oder daran befestigt ist, oder
(b) Ausrichtung der Peptide durch die Oligomerisierungsdomäne, die an jedem Peptid bereitgestellt oder daran befestigt ist.

34. Verfahren gemäß Anspruch 33, wobei die monomeren funktionellen MHC-Peptid-Komplexe in Schritt (ii) durch Rückfaltung der MHC-α- und β-Ketten in Anwesenheit des Peptids wie in Schritt (i) modifiziert bereitgestellt werden.

35. Verfahren gemäß Anspruch 33, wobei die monomeren funktionellen MHC-Peptid-Komplexe in Schritt (ii) durch Peptidaustausch für rückgefaltete funktionelle MHC-Komplexe in Anwesenheit des Peptids wie in Schritt (i) modifiziert bereitgestellt werden.

## Revendications

1. Oligomère de CMH comprenant au moins deux complexes de CMH fonctionnels possédant un sillon de liaison peptidique chaque complexe CMH possédant un peptide lié dans le sillon de liaison peptidique du complexe CMH,
dans lequel chaque peptide possède une modification qui permet, d'une manière prédéterminée, la formation spécifique et/ou sélective d'un oligomère des complexes de CMH fonctionnels par le biais d'une structure de coeur, ladite structure de coeur étant une entité permettant une liaison simultanée de chaque peptide.

2. Oligomères de CMH selon la revendication 1, dans lesquels les complexes de CMH ont été oligomérisés au niveau de leurs peptides après l'assemblage des complexes de CMH monomères fonctionnels incluant le peptide.

3. Oligomères de CMH selon la revendication 1 ou 2, dans lesquels chaque peptide comprend une modification choisie dans le groupe constitué par un site d'attachement spécifique et un domaine d'oligomérisation,
dans lequel l'oligomérisation des complexes de CMH fonctionnels se produit par le biais de
(i) la liaison de chaque peptide à une entité multivalente au niveau du site d'attachement spécifique présent sur ou attaché à chaque peptide, ou
(ii) l'alignement des peptides par le biais du domaine d'oligomérisation présent sur ou attaché à chaque peptide
dans lequel les complexes de CMH dans l'oligomère sont reliés à une structure de coeur fournie par l'entité multivalente ou par l'alignement des peptides.

4. Oligomère de CMH selon la revendication 3, dans lequel l'oligomérisation se produit par le biais d'une liaison du peptide à une entité multivalente.

5. Oligomère de CMH selon la revendication 4, dans lequel la liaison se produit par le biais d'une reconnaissance d'un site d'attachement spécifique présent sur le peptide par un site de reconnaissance présent sur l'entité multivalente.

6. Oligomère de CMH selon l'une quelconque des revendications 3 à 5, dans lequel le peptide comprend une partie de liaison au CMH et une partie séparée portant ou formant la modification.

7. Oligomère de CMH selon la revendication 6, dans lequel le peptide comprend en outre un liant polypeptidique entre sa partie de liaison au CMH et la partie séparée, le liant comprenant de préférence une longueur de 3 à 10 acides aminés.

8. Oligomère de CMH selon la revendication 5, dans lequel le site d'attachement spécifique et le site de reconnaissance sont choisis parmi les éléments d'une paire de liaisons spécifiques.

9. Oligomère de CMH selon la revendication 8, dans lequel la paire de liaisons spécifiques est choisie dans le groupe constitué par haptène/anticorps, épitope/anticorps, ligand/récepteur, substrat ou analogue d'un substrat/enzyme, co-facteur ou analogue d'un co-facteur/enzyme, acide nucléique/acide nucléique complémentaire, sucre/lectine, biotine/protéine de la famille des avidines, telle que l'avidine, la streptavidine, la neutravidine, Streptag®/Streptactine®.

10. Oligomère de CMH selon l'une quelconque des revendications 4 à 7, dans lequel l'attachement se produit par le biais d'une liaison covalente du peptide à l'entité multivalente et le site d'attachement et le site de reconnaissance sont des fragments capables de créer une liaison covalente l'un avec l'autre.

11. Oligomère de CMH selon la revendication 10, dans lequel la liaison covalente formée est d'un type choisi dans le groupe constitué par une oxime, une hydrazone, une thiazolidine, une oxazolidine, un thioéther, un disulfure et un peptide.

12. Oligomère de CMH selon l'une quelconque des revendications 4 à 11, dans lequel l'entité multivalente est choisie dans le groupe constitué par un polymère naturel ou un dérivé de celui-ci, tel qu'une protéine, un polypeptide ramifié (dendrimère), une protéine multimère, un acide nucléique, un polysaccharide tel que le dextrane, l'amidon, la cellulose, l'acide hyaluronique, la chitine ou l'acide alginique ou un dérivé de ces polysaccharides, un oligonucléotide, un oligonucléotide cyclique, un polymère synthétique, une membrane phospholipidique telle qu'une vésicule ou un liposome, et une particule inorganique.

13. Oligomère de CMH selon la revendication 12, dans lequel l'entité multivalente est un polymère naturel ou synthétique et les sites de reconnaissance sont fournis par le squelette de l'entité multivalente, ou sont attachés à celle-ci.

14. Oligomère de CMH selon la revendication 11, dans lequel l'entité multivalente est un polypeptide ramifié (dendrimère), de préférence ayant un site de reconnaissance incorporé en des sites prédéterminés dans deux de ses ramifications ou plus.

15. Oligomère de CMH selon la revendication 14, dans lequel chaque ramification du peptide comprend une longueur de moins de 24 acides aminés.

16. Oligomère de CMH selon la revendication 10, dans lequel l'entité multivalente est un oligonucléotide ou un oligonucléotide cyclique, l'oligonucléotide cyclique comprenant de préférence une longueur de moins de 150 bases de circonférence.

17. Oligomère de CMH selon la revendication 3, dans lequel l'oligomérisation se produit par le biais de l'alignement des peptides.

18. Oligomère de CMH selon la revendication 17, dans lequel chaque peptide comprend attaché sur celui-ci un domaine d'oligomérisation choisi de préférence dans le groupe constitué par un domaine d'oligomérisation peptidique et un acide nucléique.

19. Oligomère de CMH selon la revendication 18, dans lequel le domaine d'oligomérisation est un domaine d'oligomérisation peptidique qui est choisi dans le groupe constitué par un domaine d'anticorps constant, la kératine et un domaine d'oligomérisation d'une protéine à superhélice formant l'oligomère.

20. Oligomère de CMH selon la revendication 18, dans lequel, en outre, chaque peptide comprend séquentiellement deux domaines d'oligomérisation ne se chevauchant pas, et dans lequel le second domaine d'oligomérisation du n^{ème} peptide dans l'oligomère se dimérise avec le premier domaine d'oligomérisation du peptide (n+1)^{ème} dans l'oligomère.

21. Oligomère de CMH selon la revendication 20, dans lequel le second domaine d'oligomérisation du dernier peptide dans l'oligomère se dimérise avec le premier domaine d'oligomérisation du premier peptide dans l'oligomère pour former une structure cyclique.

22. Oligomère de CMH selon la revendication 20 ou 21, dans lequel les premier et second domaines d'oligomérisation sont des acides nucléiques.

23. Oligomère de CMH selon l'une quelconque des revendications précédentes, l'oligomère de CMH ne comprenant sensiblement pas de sections de peptide se liant au CMH qui ne sont pas liées à un complexe peptide-CMH.

24. Oligomère de CMH selon l'une quelconque des revendications précédentes, dans lequel le complexe de CMH est dérivé de la partie extracellulaire d'un complexe de CMH de classe I.

25. Oligomère de CMH selon l'une quelconque des revendications précédentes, dans lequel le complexe de CMH est dérivé de la partie extracellulaire d'un complexe de CMH de classe II.

26. Oligomère de CMH selon l'une quelconque des revendications précédentes, dans lequel les peptides sont essentiellement homogènes dans leur partie de liaison au CMH.

27. Oligomère de CMH selon l'une quelconque des revendications précédentes, dans lequel au moins l'un des complexes de CMH fonctionnels monomères est CD1 et le peptide correspondant est un lipide.

28. Oligomère de CMH selon l'une quelconque des revendications précédentes, dans lequel essentiellement aucune des chaînes latérales d'acides aminés de la partie de liaison au CMH du peptide et/ou des chaînes alpha ou bêta du CMH dans les complexes peptide-CMH compris dans l'oligomère n'a été modifiée dans le procédé d'oligomérisation.

29. Composition pharmaceutique ou de diagnostic comprenant un oligomère de CMH selon l'une quelconque des revendications précédentes, facultativement en combinaison avec un excipient pharmaceutiquement acceptable.

30. Méthode de marquage et/ou de détection des lymphocytes T de mammifère selon la spécificité de leur récepteur d'antigène, la méthode comprenant :
(i) la combinaison d'un oligomère de CMH selon l'une quelconque des revendications 1 à 28 et d'une suspension ou d'un échantillon biologique comprenant des lymphocytes T, et
(ii) la détection de la présence d'une liaison spécifique dudit complexe et des lymphocytes T.

31. Méthode de séparation des lymphocytes T de mammifère selon la spécificité de leur récepteur d'antigène, la méthode comprenant :
(i) la combinaison d'un oligomère de CMH selon l'une quelconque des revendications 1 à 28 et d'une suspension ou d'un échantillon biologique comprenant des lymphocytes T, et
(ii) la séparation des lymphocytes T liés audit complexe des cellules non liées.

32. Méthode selon la revendication 30 ou 31, dans laquelle à la place des lymphocytes T, un type de lymphocyte est marqué, détecté ou séparé selon un type spécifique de molécule de surface cellulaire qui se lie spécifiquement à une molécule du CMH.

33. Méthode de formation d'un oligomère de CMH tel que défini selon l'une quelconque des revendications 1 à 28 et comprenant au moins deux complexes de CMH fonctionnels possédant un sillon de liaison peptidique, chaque complexe de CMH comprenant un peptide lié dans le sillon de liaison peptidique du complexe de CMH, chaque peptide ayant une modification qui permet une oligomérisation très spécifique des complexes de CMH fonctionnels par le biais d'une structure de coeur, ladite méthode comprenant les étapes consistant à :
(i) fournir un ou plusieurs peptides susceptibles de liaison dans le sillon peptidique de chaque complexe du CMH fonctionnel, ledit peptide étant modifié par
(a) la fourniture d'un site d'attachement spécifique, ou
(b) la fourniture d'un domaine d'oligomérisation ;
(ii) utiliser des complexes peptide-CMH fonctionnels monomères incluant le peptide modifié en (i) ; et
(iii) oligomériser les complexes de CMH fonctionnels en
(a) fournissant une entité multivalente et liant lesdits complexes peptide-CMH fonctionnels monomères à l'entité multivalente au niveau du site d'attachement spécifique présent sur ou attaché à chaque peptide, ou
(b) alignant les peptides par le biais du domaine d'oligomérisation présent sur chaque peptide ou attaché à chaque peptide.

34. Méthode selon la revendication 33, dans laquelle les complexes peptide-CMH fonctionnels monomères sont fournis dans l'étape (ii) par repliage des chaînes a et β du CMH en présence du peptide comme modifié dans l'étape (i).

35. Méthode selon la revendication 33, dans laquelle les complexes peptide-CMH fonctionnels monomères sont fournis dans l'étape (ii) par échange peptidique pour les complexes de CMH fonctionnels repliés en présence du peptide comme modifié dans l'étape (i).
